# EUROPÄISCHE PATENTSCHRIFT

(11) **EP 1 242 437 B1**
(45) Veröffentlichungstag und Bekanntmachung des Hinweises auf die Patenterteilung: **03.11.2010**
(21) Anmeldenummer: 00983221.3
(22) Anmeldetag: 05.12.2000
(51) Int. Cl.: C07H 7/04, A61K 31/70, A61P 19/00, C12P 19/02

(54) **NEUE STROMELYSIN INHIBITOREN**
NOVEL STROMELYSIN INHIBITORS
NOUVEAUX INHIBITEURS DE LA STROMELYSINE

(30) Priorität: 16.12.1999 DE 19960640
(43) Veröffentlichungstag der Anmeldung: 25.09.2002
(73) Patentinhaber: Sanofi-Aventis Deutschland GmbH, 65929 Frankfurt am Main (DE)
(72) Erfinder: HOPMANN, Cordula, 60322 Frankfurt am Main (DE); KNAUF, Martin Albert, 6037 Root LV (CH); WEITHMANN, Klaus-Ulrich, 65719 Hofheim (DE); WINK, Joachim, 63322 Rödermark (DE)
(86) Internationale Anmeldenummer: PCT/EP2000/012208
(87) Internationale Veröffentlichungsnummer: WO 2001/044264

(56) Entgegenhaltungen:
- WO-A-97/40031
- WO-A-98/31697
- US-A- 5 968 795
- PATENT ABSTRACTS OF JAPAN vol. 1996, no. 11, 29. November 1996 (1996-11-29) & JP 08 175990 A (MITSUBISHI CHEM CORP), 9. Juli 1996 (1996-07-09)
- YASUZAWA T. ET AL.: "Structures of KS-501 and KS-502, the new inhibitors of Ca2+ and calmodulin-dependent cyclic nucleotide phosphodiesterase" THE JOURNAL OF ANTIBIOTICS, Bd. XLIII, Nr. 4, 1990, Seiten 336-343, XP002170090 in der Anmeldung erwähnt

## Beschreibung

Die Erfindung betrifft Stromemycin, Stromemycinderivate, Verfahren zu deren Herstellung und Verwendung derselben als Arzneimittel und Stromelysin Inhibitoren.

Stromelysin (Matrix Metalloproteinase 3) ist eine Matrix Metalloproteinase, die als Enzym beim Abbau der Proteoglykane, die wichtige Bestandteile des Knorpelgewebes sind, maßgeblich beteiligt ist (A. J. Fosang et al. J. Clin. Invest. 98 (1996) 2292-2299). Verbindungen, die dem Stromemycin strukturell ähnlich sind, wurden von Yasuzawa et al. (The Journal of Antibiotics, Band XLIII, Nr. 4, (1990), Seiten 336 - 343) beschrieben.

Inhibitoren von Matrix Metallproteinasen sind in

WO 97/40031 beschrieben.

In dem Bestreben, wirksame Verbindungen zur Behandlung von Bindegewebserkrankungen zu finden, wurde nun gefunden, dass das erfindungsgemäße Stromemycin und die Stromemycinderivate Inhibitoren der Matrix Metalloproteinase Stromelysin sind.

Die Erfindung betrifft daher die Verbindung der Formel I 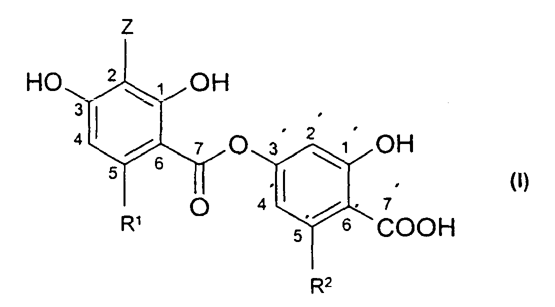 und/oder eine stereoisomere Form der Verbindung der Formel und/oder ein physiologisch verträgliches Salz der Verbindung der Formel I, wobei
- R¹ für: 1. (C₃ - C₁₀)-Alkyl, worin Alkyl gerade oder verzweigt ist und unsubstituiert oder ein-, zwei- oder dreifach substituiert ist durch
   1.1 -OR³, worin R³ Wasserstoffatom oder (C₁-C₄)-Alkyl bedeutet,
   1.2 -NR⁴R⁵, worin R⁴ und R⁵ unabhängig voneinander Wasserstoffatom oder (C₁-C₄)-Alkyl bedeuten,
   1.3 Halogen wie -F, -Cl, -Br oder -I,
   1.4 =O oder
   1.5 -COOH, oder
2. (C₃ - C₁₀)-Alkenyl, worin Alkenyl gerade oder verzweigt ist und unsubstituiert oder ein-, zwei- oder dreifach substituiert ist durch
   2.1 -OR³, worin R³ Wasserstoffatom oder (C₁-C₄)-Alkyl bedeutet,
   2.2 -NR⁴R⁵, worin R⁴ und R⁵ unabhängig voneinander Wasserstoffatom oder (C₁-C₄)-Alkyl bedeuten,
   2.3 Halogen wie -F, -Cl, -Br oder -I,
   2.4 =O oder
   2.5 -COOH. steht.
- R² für: 1. (C₃ - C₁₀)-Alkyl, worin Alkyl gerade oder verzweigt ist und unsubstituiert oder ein-, zwei- oder dreifach substituiert ist durch
   1.1 -OR³, worin R³ Wasserstoffatom oder (C₁-C₄)-Alkyl bedeutet,
   1.2 -NR⁴R⁵, worin R⁴ und R⁵ unabhängig voneinander Wasserstoffatom oder (C₁-C₄)-Alkyl bedeuten,
   1.3 Halogen wie -F, -Cl, -Br oder -I,
   1.4 =O oder
   1.5 -COOH, oder
2. (C₃ - C₁₀)-Alkenyl, worin Alkenyl gerade oder verzweigt ist und unsubstituiert oder ein-, zwei- oder dreifach substituiert ist durch
   2.1 -OR³, worin R³ Wasserstoffatom oder (C₁-C₄)-Alkyl bedeutet,
   2.2 -NR⁴R⁵, worin R⁴ und R⁵ unabhängig voneinander Wasserstoffatom oder (C₁-C₄)-Alkyl bedeuten,
   2.3 Halogen wie -F, -Cl, -Br oder -I,
   2.4 =O oder
   2.5 -COOH, steht, und
- Z für: eine Hexose steht, wobei der Zucker in pyranoider Form über eine C-glycosidische Bindung verknüpft ist.

Bevorzugt ist eine Verbindung der Formel I, wobei
R¹ für Nonadienyl steht,
R² für Nonadienyl steht und
Z für D(+)-Glucose, D(+)-Mannose, D(+)-Galactose oder D(+)-Talose steht.

Insbesondere bevorzugt ist

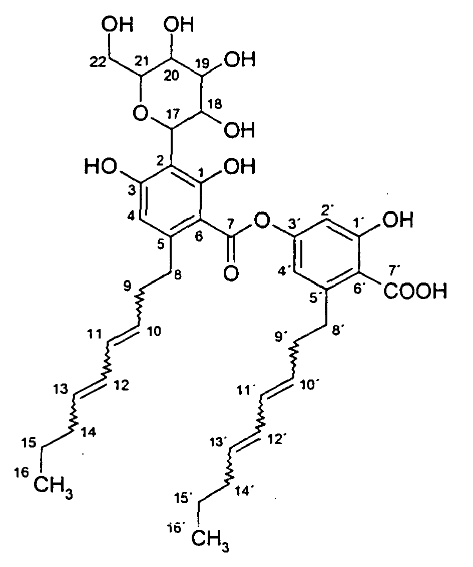

Diese Verbindung wird im folgenden als Stromemycin bezeichnet.

Mit dem Begriff Hexose werden alle in der Natur vorkommenden Hexosen der allgemeinen Formel C₆H₁₂O₆ verstanden, beispielsweise D(+)-Glucose, D(+)-Mannose, D(+)-Galactose oder D(+)-Talose.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man
a) den Mikroorganismus DSM 12038 oder dessen Mutanten oder Varianten in einem wässrigen Nährmedium kultiviert und die Verbindung Stromemycin isoliert und reinigt, oder
b) Stromemycin durch reduktive Hydrierung in eine Verbindung der Formel I überführt, worin R¹ und R² für C₉-Alkyl steht, oder
c) Stromemycin durch Ozonolyse in eine Verbindung der Formel I überführt, worin R¹ und R² für
   1.) -CH₂CH₂CH₂-OH,
   2.) -CH₂CH₂CHO oder
   3.) -CH₂CH₂COOH
   steht, oder
d) eine nach dem Verfahren c)2) hergestellte Verbindung der Formel I durch Umsetzung mit Alkylidenphosphoranen um 1 bis 7 Methylengruppen verlängert, wobei die eingeführten Seitenketten funktionelle Gruppen wie Hydroxy-, Ether-, Amino-Gruppen oder F-, Cl-, Br- oder I-Reste besitzen können.
e) eine nach den Verfahren a), b), c) oder d) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
f) die nach den Verfahren a), b), c), d) oder e) hergestellt Verbindung der Formel I entweder in freier Form isoliert oder im Falle des Vorliegens von sauren oder basischen Gruppen in physiologisch verträgliche Salze umwandelt.

Der Mikroorganismus DSM 12038 gehört zur Gruppe der Pilze und wurde am 4. März 1998 unter den Bedingungen des Budapester Vertrags bei der Deutschen Sammlung für Mikroorganismen und Zellkulturen, Mascheroder Weg 1b, D-38124 Braunschweig unter der Nummer DSM 12038 hinterlegt.

Unter Varianten von DSM 12038 werden Stämme von DSM 12038 verstanden, die durch Vereinzelung aus einer Kultur von DSM 12038 gewonnen wurden, soweit sie Stromemycin herstellen. Unter Mutanten von DSM 12038 werden Stämme von DSM 12038 verstanden, die nach Mutation aus einer Kultur von DSM 12038 gewonnen wurden, soweit sie Stromemycin herstellen. Mutanten von DSM 12038 können in an sich bekannter Weise durch physikalische Mittel, beispielsweise Bestrahlung wie Ultraviolett- oder Röntgenstrahlung oder durch chemische Mutagene, beispielsweise Ethylmethansulfonat (EMS); 2-Hydroxy-4-methoxy-benzophenon (MOB) oder N-Methyl-N'-nitro-N-nitrosoguanidin (MNNG) erzeugt werden.

Das Auffinden der Mutanten erfolgt beispielsweise durch Probennahme aus dem Kultivierungsmediums und Bestimmung der inhibierenden Wirkung auf das Stromelysin. Die Herstellung von Stromemycin erfolgt durch Kultivierung von DSM 12038. Die Nährlösung enthält Kohlenstoffquellen wie Saccharose, Maisstärke, Dextrose, Lactose, D-Mannit, Molasse oder Malzextrakt und Stickstoffquellen wie Sojabohnenmehl, Erdnußmehl, Proteine, Peptone, Peptide, Tryptone, Fleischextrakt, Hefeextrakt oder Ammoniumsalze oder Nitrate.

Die Nährlösung enthält auch anorganische Salze wie Natriumhydrogenphosphat, Natriumchlorid, Calciumchlorid, Calciumsulfat, Calciumcarbonat, Magnesiumsulfat oder Kaliumhydrogenphosphat. Ferner kann dem Nährmedium auch Fett wie Ölsäuremethylester oder Sojaöl zugesetzt werden. Daneben werden auch Spurenelemente wie Eisen-, Mangan-, Kupfer-, Zink-, Kobalt- oder andere Metallsalze zugegeben.

Eine bevorzugte Nährlösung enthält etwa von 0,1 % bis 2 % Caseinpepton, vorzugsweise von 0,3 % bis 1 %, von etwa 0,1 % bis 2 % Fleischpepton, vorzugsweise von 0,3 % bis 2 %, von 0,5 % bis 5 % Glucose, vorzugsweise von 0,5 % bis 2 %, und von 0,5 % bis 5 % Maltose, vorzugsweise von 0,3 % bis 2 %. Die Prozentangaben beziehen sich auf das Gewicht der gesamten Nährlösung.

Die Kultivierung von DSM 12038 erfolgt bei Temperaturen von 20 °C bis 35 °C, bevorzugt bei 23 °C bis 28°C und bei pH-Werten von 5 bis 9, vorzugsweise bei Werten von 4 bis 6. Die Kultivierung erfolgt aerob zunächst im Schüttelkolben und danach im Fermenter unter Rühren und Belüftung mit Luft oder reinem Sauerstoff. Die Kultivierung der Mikroorganismen in den Fermentern erfolgt über einen Zeitraum von 48 bis 240 Stunden, bevorzugt von 15 bis 72 Stunden, insbesondere von 15 bis 30 Stunden.

Die Bildung von Stromemycin erreicht ihr Maximum nach etwa 15 bis 30 Stunden.

Die Isolierung von Stromemycin erfolgt direkt aus der Nährlösung oder nach Abtrennung der Zellen beispielsweise durch Zentrifugation oder Filtration. Die Isolierung von Stromemycin kann durch Extraktion mit Lösungsmitteln oder durch Adsorption an Harze wie XAD 16, HP 20, MCI Gel® CHP20P oder Ionenaustauscher erfolgen.
Die Reinigung erfolgt beispielsweise durch Chromatographie an Adsoptionsharzen wie an Diaion^{®} HP-20 (Mitsubishi Casei Corp., Tokyo), an Amberlite^{®} XAD 7 (Rohm and Haas, USA), an Amberchrom^{®} CG (Toso Haas, Philadelphia, USA). Die Trennungen können in einem weiten pH-Bereich durchgeführt werden. Bevorzugt ist der Bereich von pH 1 bis pH 9, besonders bevorzugt von pH 2 bis pH 8. Geeignet sind darüber hinaus Reverse Phase-Träger, die im Rahmen der Hochdruckflüssigkeits-Chromatographie (HPLC) eingesetzt werden. Ein weiteres Isolierungsverfahren ist die Verwendung von Molekularsieben wie Fractogel® TSK HW-40S oder Sephadex® LH-20.

In dem C₉-Alkenylrest (Nonadienylseitenkette) können ein oder zwei Doppelbindungen vorkommen, bevorzugt zwei, die in cis- und/oder in trans-Konfiguration vorliegen. Bei dem Zuckerrest sind alle entsprechend C-glycosidisch verknüpften C₆-Stereoisomere möglich.

Als Ausgangsstoff für die Herstellung der Stromemycinderivate dient das mikrobiologisch hergestellte Stromemycin. Die Herstellung der Verbindungen der Formel I, worin R¹ und/oder R² für C₉-Alkyl steht erfolgt durch reduktive Hydrierung von Stromemycin in an sich bekannter Weise, beispielsweise wie beschrieben bei P. N. Rylander in "Hydrogenation Methods", Academic Press, New York (1985), Kapitel 2. Durch Umsetzung mit Reagenzien wie OsO₄ ist es möglich die Doppelbindungen in den Seitenketten zu hydroxylieren (siehe in Chem. Rev. 80, 187 (1980)).

Durch Ozonolyse der Doppelbindung in den Nonadienylseitenketten (R¹ und R²) entstehen bekanntlich entsprechende Derivate mit C₃-Seitenketten, die je nach oxidativer oder reduktiver Aufarbeitung Aldehyd- (z. B. mit Zn/Essigsäure oder Dimethylsulfid/Methanol), Carboxyl- (z. B. mit H₂O₂) oder OH-Gruppen (z. B. mit LiAlH₄ oder NaBH₄), als funktionelle Gruppen tragen (W. Carruthers, "Some Modern Methods of Organic Synthesis", Cambridge University Press (1971), Ch. 6; White, King und O'Brien, Tetrahedron Lett. 3591 (1971); Bailey, P. S., "Ozonisation in Organic Chemistry", Vol.1 and Vol.2, New York, Academic Press (1978, 1982)).
Durch Umsetzung der Aldehydderivate mit Alkylidenphosphoranen mittels der bekannten Wittig-Reaktion lassen sich anschließend die C₃-Ketten unter milden Bedingungen wieder verlängern, z. B. zu einer Länge von 4 bis 10 Kohlenstoffatomen, wobei die eingeführten Seitenketten funktionelle Gruppen, z. B. OR³ (R³ = H oder Alkylrest mit 1 bis 4 Kohlenstoffatomen), NR⁴R⁵ (R⁴, R⁵ = H oder Alkylrest mit 1 bis 4 Kohlenstoffatomen), F, Cl, Br, I beinhalten können, wie beschrieben in: H. J. Bestmann et al., "Selected Topics of the Wittig Reaction in the Synthesis of Natural Products" Topics in Current Chemistry 109, 85, (1983).

Die Modifikation der Zucker in der Verbindung der Formel I erfolgt nach bekannten Verfahren (H. Paulsen, Angew. Chem. Int. Ed. 21 (1982) S. 155; R.R. Schmidt, Angew. Chem. Int. Ed. 25 (1986) S. 212-235; T. Ogawa, Tetrahedron Lett. 31 (1990) 2439 - 2442).

Unter pharmakologisch verträglichen Salzen der Verbindung der Formel I versteht man sowohl anorganische als auch organische Salze, wie sie in Remington's Pharmaceutical Sciences (17. Auflage, Seite 1418 (1985)) beschrieben sind. Die Herstellung physiologisch verträglicher Salze aus zur Salzbildung befähigten Verbindungen der Formel I, einschließlich deren stereoisomeren Formen, erfolgt in an sich bekannter Weise. Die Carbonsäure bildet mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindung der Formel I basische Gruppen aufweist, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Benzolsulfon-, Phosphor-, Methansulfon-, p-Toluolsulfon-, 4-Brombenzol-sulfon-, Trifluormethylsulfon-, Cyclohexylamidosulfon-, Essig-, Oxal-, Wein-, Bernstein- und Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von Matrix-abbauenden Enzymen wie Matrix Metalloproteinase Stromelysin, beteiligt ist. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie und septischem Schock.

Die erfindungsgemäßen Arzneimittel werden im allgemeinen oral oder parenteral verabreicht. Die rektale, inhalative, nasale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylen-glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

### Beispiel 1 Herstellung einer Sporensuspension von DSM 12038

100 mL Nährlösung (20 g Malzextrakt, 2 g Hefeextrakt, 10 g Glucose, 0,5 g (NH₄)₂HPO₄ in 1 L Leitungswasser, pH-Wert vor der Sterilisation: 6.0) in einem 500 mL sterilen Erlenmeyerkolben wurden mit dem Stamm DSM 12038 beimpft und 72 Stunden bei 25 °C und 140 UpM auf einer rotierenden Schüttelmaschine inkubiert. Dann wurden 120 mL Kulturflüssigkeit in einem sterilen 500 mL Erlenmeyerkolben mit dem Nährboden Hafermehlinfus, 2.0 g/L, dem zusätzlich 15 g Agar/L zur Verfestigung zugegeben wurden, gleichmäßig verteilt und mit der Schüttelkultur beimpft. Der Stamm wurde 10 bis 14 Tage bei 25 °C inkubiert. Die vom Stamm DSM 12038 gebildeten Sporen wurden mit 500 mL entionisiertem Wasser, welches einen Tropfen Triton^{®} X 100 (Firma Serva) enthielt, abgeschwemmt, sofort weiterverwendet oder bei -22 °C in 50 % Glycerin oder in 10 % Dimethylsulfoxid bei -140 °C aufbewahrt.

### Beispiel 2: Herstellung einer Schüttelkultur

Ein steriler 500 mL Erlenmeyerkolben mit 100 mL einer Nährlösung enthaltend 0,5 % Caseinpepton, 0,5 % Fleischpepton, 1 % Glucose und 10 % Maltose wurde mit 0,2 mL Sporensuspension, hergestellt gemäß Beispiel 1, beimpft und auf einer Schüttelmaschine unter Ausschluß von Licht bei 140 UpM und 25 °C inkubiert. Die maximale Menge von Stromemycin wurde nach etwa 27 Stunden erreicht.
Zum Animpfen von 10 L oder 100 L Fermentern wurde eine 72 h alte Schüttelkultur, enthaltend den Stamm DSM 12038 und eine Nährlösung aus 2 % Malzextrakt, 0,2 % Hefeextrakt, 1 % Glucose und 0,05 % Ammoniumhydrogenphosphat verwendet (Animpfmenge etwa 5 %).

### Beispiel 3 Herstellung von Stromemycin

Ein 10 L Fermenter wurde unter folgenden Bedingungen betrieben:

| | | |
|---|---|---|
| Nährmedium: | Caseinpepton | 5 g/L |
| | Fleischpepton | 5 g/L |
| | Glucose | 10 g/L |
| | Maltose | 10 g/L |
| Inkubationszeit: | 25 bis 30 Stunden | |
| Inkubationstemperatur: | 25 °C | |
| Rühreregeschwindigkeit: | 300 UpM | |
| Belüftung: | 0,5 L/min | |
| pH | 5 ± 0.5 | |

Durch Zugabe von 1 bis 2 mL ethanolischer Polyollösung konnte die Schaumbildung unterdrückt werden. Das Produktionsmaximum wurde nach 27 Stunden erreicht.

### Beispiel 4: Isolierung von Stromemycin

7,5 L der nach Beispiel 3 gewonnen Kulturlösung wurden abzentifugiert und das Kulturfiltrat im Batchverfahren auf 1,5 L Adsorptionsharz MCI Gel^{®} CHP20P adsorbiert. Das Harz wurde auf eine Nutsche gegeben und zunächst mit 4 L H₂O gewaschen. Anschließend erfolgte die Elution mit jeweils 2 L Fraktionen eines Isopropanol/Wasser Gemisches, enthaltend jeweils 20%, 40%, 70% oder 100 % Isopropanol. Nach HPLC Prüfung und Aktivitätstest wurde Stromemycin mit den ersten beiden Fraktionen (20% und 40% Isopropanol) eluiert. Die Fraktionen wurden gesammelt und gefriergetrocknet. Das Produkt wurde anschließend mittels HPLC weiter aufgereinigt:
1.) Säule: RP18, Nucleosil 100 RP18-AB (Macherey & Nagel), 250 x 21 mm, 5µ
   Eluant: CH₃CN / 0.1 %TFA
   Gradient: 37 min 40% CH₃CN isokratisch, anschließend in 10 min auf 100% CH₃CN. Fluß: 9 ml/ min; Detektion : 275 nm
   Stromemycin eluierte nach 52 min.
2.) Säule: Fractogel TSK HW40S, 200ml (Erimatechnik), 300 x 25 mm
   Eluant: MeOH; Fluß: 2 ml/min; Detektion: 230 nm
   Stromemycin eluierte nach 75 min.
   Ausbeute: 25 mg aus 7.5 L Fermentermedium.

Das gewonne Stromemycin hat folgende Eigenschaften:

| | |
|---|---|
| Aussehen: | farbloser Feststoff; löslich in Methanol, Dimethylsulfoxid (DMSO); stabil in neutralem Milieu, jedoch unbeständig in stark-saurer und alkalischer Lösung. |
| Summenformel: | C₃₈H₄₈O₁₂ |
| HPLC (High Pressure | |
| Liquid Chromatography): | Säule: Purospher RP-18e (125 x 3 mm, 5µ) |
| | Eluant: Gradient: CH₃CN / 0,1% H₃PO₄ in 20 min von 0% auf 100% CH3_{C}N |
| | Fluß: 0,6 ml/min |
| | Retentionszeit: 16,4 min |
| | Detektion: 230 nm |
| Molekulargewicht: | 696,8 Da |
| HR-FAB-MS: | 697,32332 [M+H]⁺ |
| ¹H-und ¹³C-NMR: | siehe Tabelle 1 |
| UV/VIS: | MeOH λₙₘ(log ε): 216 nm (4.87), 267 (4.33), 304 (4.34) |
| FT-IR: | (KBr), v=3419 cm⁻¹(br), 2929 (m), 1608 (s), 1434 (w), 1252 (m), 1204 (w), 1151 (m), 1084 (w). |

**Tabelle 1: ¹H- und ¹³C-chemische Verschiebungen von Stromemycin in DMSO-d₆, ppm rel. TMS, 300K**

| Position | ¹H [ppm] | ¹³C [ppm] |
|---|---|---|
| 1 | - | 158.29 |
| 1-OH | 9.70 | - |
| 2 | - | 110.56 |
| 3 | - | 159.32 |
| 3-OH | 9.81 | - |
| 4 | 6.33 | 109.58 |
| 5 | - | 142.46 |
| 6 | - | 108.83 |
| 7 | - | 167.36 |
| 8 | 2.75 | 34.27 |
| 9 | 2.32 | 34.02 |
| 10 | 5.58 | 130.79 |
| 11 | 6.0 | 130.72 (130.79)^{a)} |
| 12 | 6.0 | 130.24 (130.31)^{a)} |
| 13 | 5.55 | 132.22 (132.38)^{a)} |
| 14 | 1.99 | 34.02 |
| 15 | 1.34 | 21.96 (21.97)^{a)} |
| 16 | 0.85 | 13.50 |
| 17 | 4.70 | 74.40 |
| 18 | 3.68 | 71.53 |
| 19 | 3.24 | 78.38 |
| 20 | 3.27 | 69.65 |
| 21 | 3.24 | 81.21 |
| 22 | 3.54/3.66 | 60.51 |
| 1' | - | 157.17 |
| 1'-OH | 10.67 ^{c)} | - |
| 2' | 6.63 | 107.31 |
| 3' | - | 151.55 |
| 4' | 6.56 | 113.18 |
| 5' | - | 142.25 |
| 6' | - | 118.40 |
| 7' | - | 169.73 |
| 7'-COOH | n.d. | - |
| 8' | 2.71 | 33.44 |
| 9' | 2.29 | 33.61 |
| 10' | 5.58 | 130.79 |
| 11' | 6.0 | 130.79 (130.72)^{a)} |
| 12' | 6.0 | 130.31 (130.24)^{a)} |
| 13' | 5.55 | 132.38 (132.22)^{a)} |
| 14' | 1.99 | 34.02 |
| 15' | 1.34 | 21.97 (21.96)^{a)} |
| 16' | 0.85 | 13.50 |

| | | |
|---|---|---|
| ^{a}) Wegen Signalüberlagerung ist eine eindeutige Zuordnung der chemischen Verschiebungen nicht möglich. ^{b)} Nahezu identische chemische Verschiebungen der Positionen 10-16 und 10'-16' verhindern eine eindeutige Unterschiedung der HMBC-Korrelation bezüglich beider Dien-Seitenketten. ^{c)} "tentative assignment" | | |

### Pharmakologische Beispiele

### Darstellung und Bestimmung der enzymatischen Aktivität der katalytischen Domäne des humanen Stromelysins

Das Enzym -Stromelysin (MMP-3) wurde dargestellt nach Ye et al. (Biochemistry; 31 (1992) Seiten 11231-11235). Zur Messung der Enzymaktivität oder der Enzyminhibitorwirkung wurden 70 µl Pufferlösung, und 10 µl Enzymlösung mit 10 µl einer 10%igen (v/v) wässrigen Dimethylsulfoxid-Lösung, die gegebenenfalls den Enzyminhibitor enthielt, für 15 Minuten inkubiert. Nach Zugabe von 10 µl einer 10%igen (v/v) wäßrigen Dimethylsulfoxid-Lösung, die 1 mmol/l des Substrates enthielt, wurde die Enzymreaktion fluoreszenzspektroskopisch verfolgt (328 nm (ex) / 393 nm(em)).
Die Enzymaktivität wird dargestellt als Extinktionszunahme/Minute. Der IC₅₀-Wert wurde als diejenige Inhibitorkonzentration ermittelt, die jeweils zu einer 50%igen Inhibierung des Enzyms führte.

Die Pufferlösung enthielt 0,05% Brij (Sigma, Deisenhofen, Deutschland) sowie 0,1 mol/l Tris/HCl, 0,1 mol/l NaCl, 0,01 mol/l CaCl₂ und 0,1 mol/l Piperazin-N,N'-bis[2-ethansulfonsäure] (pH=6,5).
Die Enzymlösung enthielt 5 µg/ml einer der nach Ye et al. dargestellten Enzymdomänen. Die Substratlösung enthielt 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland).

Der IC₅₀-Wert für Stromemycin wurde mit 115 µM bestimmt.

## Patentansprüche

1. Verbindung der Formel I 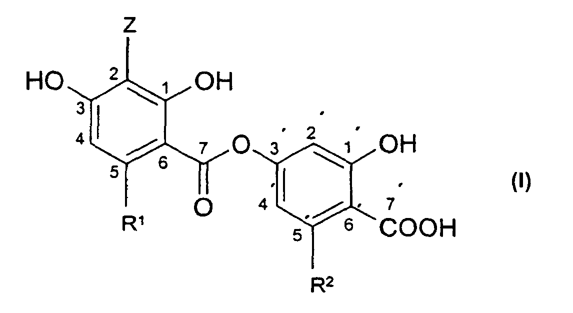 oder eine stereoisomere Form der Verbindung der Formel I oder ein physiologisch verträgliches Salz der Verbindung der Formel I, wobei
R¹ für
1. (C₃ - C₁₀)-Alkyl, worin Alkyl gerade oder verzweigt ist und unsubstituiert oder ein-, zwei- oder dreifach substituiert ist durch
1.1 -OR³, worin R³ Wasserstoffatom oder (C₁-C₄)-Alkyl bedeutet,
1.2 -NR⁴R⁵, worin R⁴ und R⁵ unabhängig voneinander Wasserstoffatom oder (C₁-C₄)-Alkyl bedeuten,
1.3 Halogen wie -F, -Cl, -Br oder -I,
1.4 =O oder
1.5 -COOH, oder
2. (C₃ - C₁₀)-Alkenyl, worin Alkenyl gerade oder verzweigt ist und unsubstituiert oder ein-, zwei- oder dreifach substituiert ist durch
2.1 -OR³, worin R³ Wasserstoffatom oder (C₁-C₄)-Alkyl bedeutet,
2.2 -NR⁴R⁵, worin R⁴ und R⁵ unabhängig voneinander Wasserstoffatom oder (C₁-C₄)-Alkyl bedeuten,
2.3 Halogen wie -F, -Cl, -Br oder -I,
2.4 =O oder
2.5 -COOH, steht,
R² für
1. (C₃ - C₁₀)-Alkyl, worin Alkyl gerade oder verzweigt ist und unsubstituiert oder ein-, zwei- oder dreifach substituiert ist durch
1.1 -OR³, worin R³ Wasserstoffatom oder (C₁-C₄)-Alkyl bedeutet,
1.2 -NR⁴R⁵, worin R⁴ und R⁵ unabhängig voneinander Wasserstoffatom oder (C₁-C₄)-Alkyl bedeuten,
1.3 Halogen wie -F, -Cl, -Br oder -I,
1.4 =O oder
1.5 -COOH, oder
2. (C₃ - C₁₀)-Alkenyl, worin Alkenyl gerade oder verzweigt ist und unsubstituiert oder ein-, zwei- oder dreifach unabhängig voneinander substituiert ist durch
2.1 -OR³, worin R³ Wasserstoffatom oder (C₁-C₄)-Alkyl bedeutet,
2.2 -NR⁴R⁵, worin R⁴ und R⁵ unabhängig voneinander Wasserstoffatom oder (C₁-C₄)-Alkyl bedeuten,
2.3 Halogen wie -F, -Cl, -Br oder -I,
2.4 =O oder
2.5 -COOH, steht, und
Z für Hexose steht, wobei die Hexose in pyranoider Form über eine C- glycosidische Bindung gebunden ist.

2. Verbindung der Formel I, gemäß Anspruch 1, **dadurch gekennzeichnet, dass**
R¹ für Nonadienyl steht,
R² für Nonadienyl steht und
Z für D(+)-Glucose, D(+)-Mannose, D(+)-Galactose oder D(+)-Talose steht.

3. Verbindung der Formel I gemäß Anspruch 1, **dadurch gekennzeichnet, dass** es die folgende Struktur hat

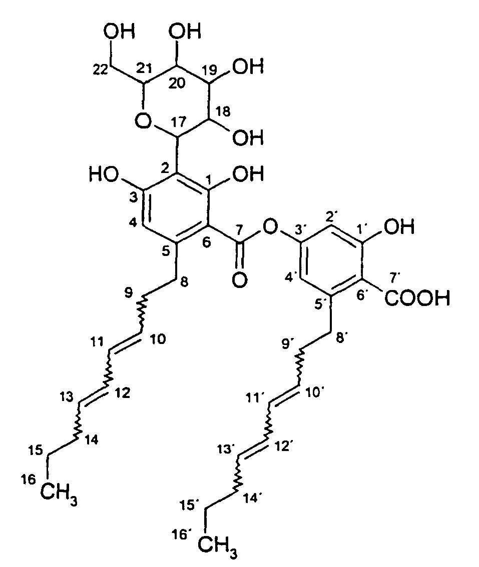

4. Verfahren zur Herstellung der Verbindung der Formel I, gemäß einem oder mehreren der Ansprüche 1 bis 3, **dadurch gekennzeichnet, daß** man
a) den Mikroorganismus DSM 12038 oder dessen Mutanten oder Varianten in einem wässrigen Nährmedium kultiviert und die Verbindung Stromemycin isoliert und reinigt, oder
b) Stromemycin durch reduktive Hydrierung in eine Verbindung der Formel I überführt, worin R¹ und R² für C₉-Alkyl steht, oder
c) Stromemycin durch Ozonolyse in eine Verbindung der Formel I überführt, worin R¹ und R² für
1.) -CH₂CH₂CH₂-OH,
2.) -CH₂CH₂CHO oder
3.) -CH₂CH₂COOH steht, oder
d) eine nach dem Verfahren c)2) hergestellte Verbindung der Formel I durch Umsetzung mit Alkylidenphosphoranen um 1 bis 7 Methylengruppen verlängert, wobei die eingeführten Seitenketten funktionelle Gruppen wie Hydroxy-, Ether-, Amino-Gruppen oder F-, Cl-, Br- oder I-Reste besitzen können.
e) eine nach den Verfahren a), b), c) oder d) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
f) die nach den Verfahren a), b), c), d) oder e) hergestellte Verbindung der Formel I entweder in freier Form isoliert oder im Falle des Vorliegens von sauren oder basischen Gruppen in physiologisch verträgliche Salze umwandelt.

5. Arzneimittel, **gekennzeichnet durch** einen wirksamen Gehalt an mindestens einer Verbindung der Formel I gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

6. Verwendung von mindestens einer Verbindung der Formel I gemäß einem oder mehreren der Ansprüche 1 bis 3, zur Herstellung von Arzneimitteln zur Prophylaxe und Therapie von Erkrankungen, an deren Verlauf eine verstärkte Aktivität von Matrix-abbauenden Metalloproteinasen beteiligt ist.

7. Verwendung gemäß Anspruch 6, zur Behandlung von degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, der Ulceration, Atherosklerose und Stenosen, aber auch zur Behandlung von Entzündungen, Krebserkrankungen, Tumormetastasenbildung, Kachexie, Anorexie und septischem Schock.

8. Verfahren zur Herstellung eines Arzneimittels, **dadurch gekennzeichnet, daß** man mindestens eine Verbindung der Formel I gemäß einem oder mehreren der Ansprüche 1 bis 3 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula I 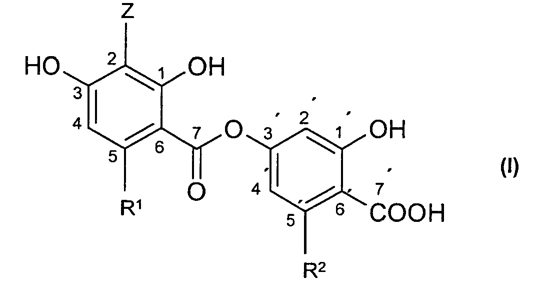 or a stereoisomeric form of the compound of the formula I or a physiologically tolerable salt of the compound of the formula I, where
R¹ is
1. (C₃-C₁₀)-alkyl, in which alkyl is linear or branched and is unsubstituted or mono-, di- or trisubstituted by
1.1 -OR³, in which R³ is a hydrogen atom or (C₁-C₄)-alkyl,
1.2 -NR⁴R⁵, in which R⁴ and R⁵ independently of one another are a hydrogen atom or (C₁-C₄)-alkyl,
1.3 halogen such as -F, -Cl, -Br or -I,
1.4 =O or
1.5 -COOH, or
2. (C₃-C₁₀)-alkenyl, in which alkenyl is linear or branched and is unsubstituted or mono-, di- or trisubstituted by
2.1 -OR³, in which R³ is a hydrogen atom or (C₁-C₄)-alkyl,
2.2 -NR⁴R⁵, in which R⁴ and R⁵ independently of one another are a hydrogen atom or (C₁-C₄)-alkyl,
2.3 halogen such as -F, -Cl, -Br or -I,
2.4 =O or
2.5 -COOH,
R² is
1. (C₃-C₁₀)-alkyl, in which alkyl is linear or branched and is unsubstituted or mono-, di- or trisubstituted by
1.1 -OR³, in which R³ is a hydrogen atom or (C₁-C₄)-alkyl,
1.2 -NR⁴R⁵, in which R⁴ and R⁵ independently of one another are a hydrogen atom or (C₁-C₄)-alkyl,
1.3 halogen such as -F, -Cl, -Br or -I,
1.4 =O or
1.5 -COOH, or
2. (C₃-C₁₀)-alkenyl, in which alkenyl is linear or branched and is unsubstituted or mono-, di- or trisubstituted by
2.1 -OR³, in which R³ is a hydrogen atom or (C₁-C₄)-alkyl,
2.2 -NR⁴R⁵, in which R⁴ and R⁵ independently of one another are a hydrogen atom or (C₁-C₄)-alkyl,
2.3 halogen such as -F, -Cl, -Br or -I,
2.4 =O or
2.5 -COOH, and
Z is a hexose, where the hexose is linked in pyranoid form via a C-glycosidic bond.

2. A compound of the formula I as claimed in claim 1, wherein
R¹ is Nonadienyl,
R² is nonadienyl and
Z is D(+)-glucose, D(+)-mannose, D(+)-galactose or D(+)-talose.

3. A compound of the formula I as claimed in claim 1, which has the following structure

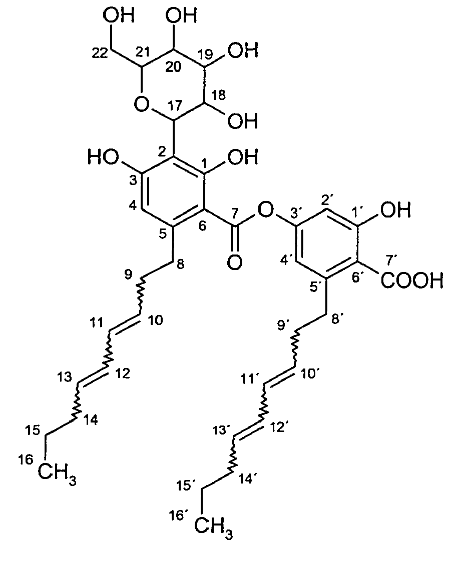

4. A process for the preparation of the compound of the formula I, as claimed in one or more of claims 1 to 3, which comprises
a) culturing the microorganism DSM 12038 or its mutants or variants in an aqueous nutrient medium and isolating and purifying the compound stromemycin, or
b) converting stromemycin by reductive hydrogenation into a compound of the formula I in which R¹ and R² are C₉-alkyl, or
c) converting stromemycin by ozonolysis into a compound of the formula I in which R¹ and R² are
1.) -CH₂CH₂CH₂-OH,
2.) -CH₂CH₂CHO or
3.) -CH₂CH₂COOH or
d) extending a compound of the formula I prepared by process c)2) by 1 to 7 methylene groups by reaction with alkylidene- phosphoranes, where the side chains introduced can have functional groups such as hydroxyl, ether, amino groups or F, Cl, Br or I radicals,
e) separating a compound of the formula I, which on account of its chemical structure occurs in enantiomeric form, prepared by process a), b), c) or d) into the pure enantiomers by salt formation with enantiomerically pure acids or bases, chromatography on chiral stationary phases or derivatization by means of chiral enantiomerically pure compounds such as amino acids, separation of the diastereomers thus obtained, and removal of the chiral auxiliary groups, or
f) either isolating the compound of the formula I prepared by process a), b), c), d) or e) in free form or, in the case of the presence of acidic or basic groups, converting it into physiologically tolerable salts.

5. A pharmaceutical which comprises an efficacious amount of at least one compound of the formula I as claimed in one or more of claims 1 to 3 together with a pharmaceutically suitable and physiologically tolerable vehicle, additive and/or other active compounds and excipients.

6. The use of at least one compound of the formula I as claimed in one or more of claims 1 to 3 for the production of pharmaceuticals for the prophylaxis and therapy of conditions whose course involves an increased activity of matrix-degrading metalloproteinases.

7. The use as claimed in claim 6, for the treatment of degenerative joint disorders such as osteoarthrosis, spondylosis, chondrolysis after joint trauma or relatively long joint immobilization after meniscus or patella injuries or tearing of the ligaments, conditions of the connective tissue such as collagenoses, periodontal conditions, wound healing disorders and chronic conditions of the locomotory apparatus such as inflammatorily, immunologically or metabolically caused acute and chronic arthritis, arthropathy, myalgia and disorders of the bone metabolism, of ulceration, atherosclerosis and stenoses, but also for the treatment of inflammation, carcinomatous disorders, formation of tumor metastases, cachexia, anorexia and septic shock.

8. A process for the production of a pharmaceutical, which comprises bringing at least one compound of the formula I as claimed in one or more of claims 1 to 3 into a suitable administration form using a pharmaceutically suitable and physiologically tolerable vehicle and, if appropriate, further suitable active compounds, additives or excipients.

## Revendications

1. Composé de formule I 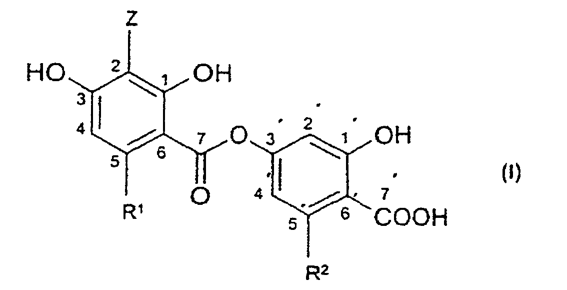 ou une forme stéréo-isomère du composé de formule I ou un sel physiologiquement acceptable du composé de formule I, où
R¹ représente
1. (C₃-C₁₀)-alkyle, où alkyle est linéaire ou ramifié et est non substitué ou monosubstitué, disubstitué ou trisubstitué par
1.1 -OR³, où R³ représente un atome d'hydrogène ou (C₁-C₄)-alkyle,
1.2 -NR⁴R⁵, où R⁴ et R⁵ signifient, indépendamment l'un de l'autre, un atome d'hydrogène ou (C₁-C₄)-alkyle,
1.3 halogène tel que -F, -Cl, -Br ou -I,
1.4 =O ou
1.5 -COOH, ou
2. (C₃-C₁₀)-alcényle, où alcényle est linéaire ou ramifié et est non substitué ou monosubstitué, disubstitué ou trisubstitué par
2.1 -OR³, où R³ représente un atome d'hydrogène ou (C₁-C₄)-alkyle,
2.2 -NR⁴R⁵, où R⁴ et R⁵ signifient, indépendamment l'un de l'autre, un atome d'hydrogène ou (C₁-C₄)-alkyle,
2.3 halogène tel que -F, -Cl, -Br ou -I,
2.4 =O ou
2.5 -COOH,
R² représente
1. (C₃-C₁₀)-alkyle, où alkyle est linéaire ou ramifié et est non substitué ou monosubstitué, disubstitué ou trisubstitué par
1.1 -OR³, où R³ représente un atome d'hydrogène ou (C₁-C₄)-alkyle,
1.2 -NR⁴R⁵, où R⁴ et R⁵ signifient, indépendamment l'un de l'autre, un atome d'hydrogène ou (C₁-C₄)-alkyle,
1.3 halogène tel que -F, -Cl, -Br ou -I,
1.4 =O ou
1.5 -COOH, ou
2. (C₃-C₁₀)-alcényle, où alcényle est linéaire ou ramifié et est non substitué ou monosubstitué, disubstitué ou trisubstitué, indépendamment l'un de l'autre, par
2.1 -OR³, où R³ représente un atome d'hydrogène ou (C₁-C₄)-alkyle,
2.2 -NR⁴R⁵, où R⁴ et R⁵ signifient, indépendamment l'un de l'autre, un atome d'hydrogène ou (C₁-C₄)-alkyle,
2.3 halogène tel que -F, -Cl, -Br ou -I,
2.4 =O ou
2.5 -COOH, et
Z représente hexose, où l'hexose est lié sous une forme pyrannoïde via une liaison C-glycosidique.

2. Composé de formule I, selon la revendication 1, **caractérisé en ce que**
R¹ représente nonadiényle,
R² représente nonadiényle et
Z représente D(+)-glucose, D(+)-mannose, D(+)- galactose ou D(+)-talose.

3. Composé de formule I, selon la revendication 1, **caractérisé en ce qu'**il présente la structure suivante

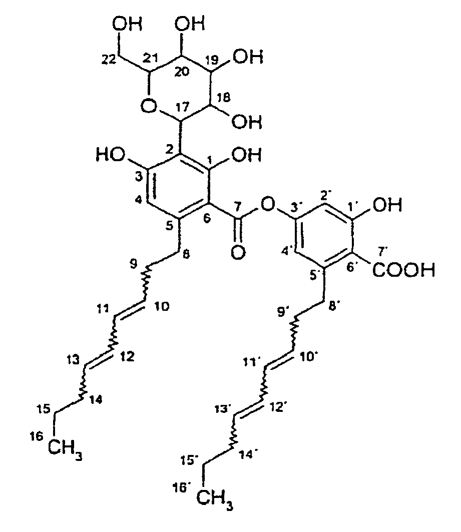

4. Procédé pour la préparation du composé de formule I selon l'une ou plusieurs des revendications 1 à 3, **caractérisé en ce qu'**on
a) cultive le microorganisme DSM 12038 ou ses mutants ou variants dans un milieu nutritif aqueux et on isole et purifie le composé stromémycine, ou
b) transforme la stromémycine par hydrogénation réductrice en un composé de formule I, où R¹ et R² représentent C₉-alkyle, ou
c) transforme la stromémycine par ozonolyse en un composé de formule I, où R¹ et R² représentent
1.) -CH₂CH₂CH₂-OH,
2.) -CH₂CH₂CHO ou
3.) -CH₂CH₂COOH, ou
d) allonge un composé de formule I préparé selon le procédé c)2) par transformation avec des alkylidène-phosphoranes de 1 à 7 groupes méthylène, où les chaînes latérales introduites peuvent présenter des groupes fonctionnels tels que des groupes hydroxy, éther, amino ou des radicaux F, Cl, Br ou I ;
e) sépare un composé de formule I, préparé selon les procédés a), b), c) ou d) qui, en raison de sa structure chimique, apparaît sous des formes énantiomères, par salification avec des acides ou des bases énantiomères pur(e)s, chromatographie sur des phases stationnaires chirales ou transformation en dérivés au moyen de composés énantiomères purs, tels que des aminoacides, séparation des diastéréo-isomères ainsi obtenus et élimination des groupes auxiliaires chiraux, en énantiomères purs, ou
f) isole le composé de formule I, préparé selon les procédés a), b), c), d) ou e) sous forme libre, ou, dans le cas de la présence de groupes acides ou basiques, convertit celui-ci en sels physiologiquement acceptables.

5. Médicament, **caractérisé par** une teneur active en au moins un composé de formule I selon l'une ou plusieurs des revendications 1 à 3 avec un support, un additif et/ou d'autres substances actives et adjuvants pharmaceutiquement approprié(s) et physiologiquement acceptable(s).

6. Utilisation d'au moins un composé de formule I selon l'une ou plusieurs des revendications 1 à 3 pour la préparation de médicaments destinés à la prophylaxie et la thérapie de maladies à l'évolution desquelles participe une activité renforcée de métalloprotéinases de dégradation de la matrice.

7. Utilisation selon la revendication 6 pour le traitement de maladies de dégénérescence des articulations, telles que les ostéoarthroses, les spondyloses, l'atrophie du cartilage après un traumatisme articulaire ou une immobilisation prolongée des articulations après des lésions au ménisque ou à la rotule ou des déchirures des ligaments, de maladies du tissu conjonctif, telles que les collagénoses, les maladies parodontales, de troubles de guérison de plaies et de maladies chroniques de l'appareil locomoteur telles que les arthrites inflammatoires, immunologiques ou métaboliques, aiguës et chroniques, les arthropathies, les myalgies et les troubles du métabolisme osseux, d'une ulcération, de l'athérosclérose et de sténoses, mais également pour le traitement d'inflammations, de maladies cancéreuses, d'une formation de métastases de tumeurs, de la cachexie, de l'anorexie et du choc septique.

8. Procédé pour la préparation d'un médicament, **caractérisé en ce qu'**on met sous une forme d'administration appropriée au moins un composé de formule I selon l'une ou plusieurs des revendications 1 à 3 avec un support pharmaceutiquement approprié et physiologiquement acceptable et le cas échéant d'autres substances actives, additifs ou adjuvants approprié(e)s.
